# EUROPEAN PATENT SPECIFICATION

(11) **EP 4 137 476 B1**
(45) Date of publication and mention of the grant of the patent: **10.07.2024**
(21) Application number: 21904615.8
(22) Date of filing: 15.07.2021
(51) Int. Cl.: C07C 209/68, C07C 211/62, C07C 209/00, C07C 68/06, C07C 69/96, C07C 209/08

(54) **METHOD FOR PREPARING ADAMANTYLTRIMETHYLAMMONIUM HYDROXIDE, AND AQUEOUS QUATERNARY AMMONIUM BASE SOLUTION PREPARED THEREBY**
VERFAHREN ZUR HERSTELLUNG VON ADAMANTYLTRIMETHYLAMMONIUMHYDROXID UND DAMIT HERGESTELLTE WÄSSRIGE LÖSUNG AUF QUARTÄRER AMMONIUMBASIS
PROCÉDÉ DE PRÉPARATION D'HYDROXYDE D'ADAMANTYLTRIMÉTHYLAMMONIUM ET SOLUTION AQUEUSE À BASE D'AMMONIUM QUATERNAIRE PRÉPARÉE PAR CE PROCÉDÉ

(30) Priority: 30.06.2021 CN 202110735794
(43) Date of publication of application: 22.02.2023
(73) Proprietor: Kente Catalysts Inc., Xianju County Taizhou City, Zhehiang 317300 (CN)
(72) Inventor: WU, Jianping, Taizhou City Zhejiang 317300 (CN); XIANG, Feiyong, Taizhou City Zhejiang 317300 (CN); LU, Jinyin, Taizhou City Zhejiang 317300 (CN); HUANG, Xiaodong, Taizhou City Zhejiang 317300 (CN); PAN, Qunyang, Taizhou City Zhejiang 317300 (CN); SHI, Xusheng, Taizhou City Zhejiang 317300 (CN); PAN, Xiaochu, Taizhou City Zhejiang 317300 (CN)
(74) Representative: JD&P Patent Attorneys Joanna Dargiewicz & Partners
(86) International application number: PCT/CN2021/106379
(87) International publication number: WO 2023/272781

(56) References cited:
- WO-A1-2016/093193
- WO-A1-2016/093193
- CN-A- 105 209 426
- CN-A- 105 209 426
- CN-A- 106 350 831
- CN-A- 107 804 855
- CN-A- 109 535 004
- US-A1- 2012 010 431
- US-A1- 2012 010 431

## Description

### TECHNICAL FIELD

The present application belongs to the technical field of molecular sieve template agent production, and more particularly, to a preparation method of adamantyltrimethylammonium hydroxide, and an aqueous quaternary ammonium base solution prepared from the same.

### BACKGROUND

With the increasingly strict requirements of environmental protection, a consumption of SSZ-13 molecular sieves used for diesel vehicle exhaust purification is increasing. As a necessary template agent for producing the SSZ-13 molecular sieves, adamantyltrimethylammonium hydroxide has a growing market demand. Researches on a preparation technology of the adamantyltrimethylammonium hydroxide are increasing.

Some researchers use adamantyldimethylamine as a raw material, carry out quaternarization on the adamantyldimethylamine with halomethane to produce adamantyltrimethylammonium halide, and then carry out ion exchange with an ion exchange resin, so that halogen ions are exchanged into hydroxide ions to obtain the adamantyltrimethylammonium hydroxide. However, the ion exchange resin is needed in this technical solution, and the ion exchange resin needs to be soaked in acid and alkali successively, and then rinsed with a large amount of water to finish the pretreatment of the ion exchange resin. On one hand, a large amount of wastewater is produced, and on the other hand, an operation is complicated and cumbersome, which is not beneficial for industrial production.

Some researchers use the adamantyltrimethylammonium halide to electrolyze, and combine adamantyltrimethylammonium cations with hydroxide ions produced by a cathode to form the adamantyltrimethylammonium hydroxide. For example, in the Chinese patent with the publication number CN110699700A, an anode plate and a cathode plate are needed during electrolysis in this technical solution, the anode plate is generally a precious metal pole plate containing platinum and iridium, which is easy to be corroded during electrolysis, and precious metals such as platinum and iridium are expensive, which is not beneficial for reducing a production cost.

In the Chinese patent with the publication number CN105209426A, adamantyl hydrochloride is used to react with formic acid and formaldehyde in a sodium hydroxide solution to prepare the adamantyldimethylamine, then the adamantyldimethylamine reacts with dimethyl carbonate at high temperature and high pressure to produce an adamantyltrimethylammonium carbonate monomethyl ester salt, and then a hydrolysis reaction is carried out on the adamantyltrimethylammonium carbonate monomethyl ester salt with calcium hydroxide to produce calcium carbonate and the adamantyltrimethylammonium hydroxide. Main defects of the above solution are as follows: (1) during the reaction of preparing the adamantyldimethylamine, sodium hydroxide is used mostly, and a content of metal ions in a product is high; (2) salification of the dimethyl carbonate with the adamantyldimethylamine is carried out at high temperature and high pressure, wherein the temperature generally exceeds 130°C, and the adamantyldimethylamine and the adamantyltrimethylammonium carbonate monomethyl ester salt are easy to decompose under high temperature to produce impurities; and (3) during reaction of the adamantyltrimethylammonium carbonate monomethyl ester salt with the calcium hydroxide, an amount of calcium hydroxide is large, and impurities such as calcium ions are easy to remain in the product, which affects a purity of the product and increases a chromaticity of the product, thus bringing certain adverse effects to a product quality of the adamantyltrimethylammonium hydroxide.

On this basis, the applicant made the present invention.

### SUMMARY

In order to reduce an impurity content in an adamantyltrimethylammonium hydroxide product and improve a product yield, the present invention aims to provide a preparation method of adamantyltrimethylammonium hydroxide in a first aspect.

The technical solutions used in the present invention are as follows.

A preparation method of adamantyltrimethylammonium hydroxide comprises the following steps of:
S1, salification: weighing dimethyladamantanamine, adding dimethyl carbonate, evenly mixing the mixture to prepare a quaternized mixture, under a helium sealing atmosphere, heating the quaternized mixture to 135°C to 150°C for reaction for no less than 6 hours, and recovering a product to obtain adamantyltrimethylammonium carbonate monomethyl ester salt, wherein a molar ratio of the dimethyladamantanamine to the dimethyl carbonate in the quaternized mixture is 1: 1 to 10; and a reaction formula is as follows: 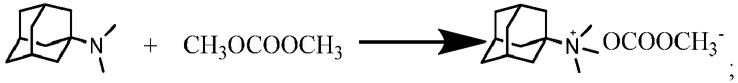
S2, hydrolysis: dissolving the adamantyltrimethylammonium carbonate monomethyl ester salt into water, adding calcium hydroxide, wherein a molar ratio of the adamantyltrimethylammonium carbonate monomethyl ester salt to the calcium hydroxide is 1: 1 to 1.045; and blowing nitrogen from a lower part of a liquid level, heating the mixture until a boiling state is reached, simultaneously removing a byproduct methanol to form the adamantyltrimethylammonium hydroxide, and recovering a product to prepare an aqueous quaternary ammonium base solution, wherein a reaction formula is as follows:

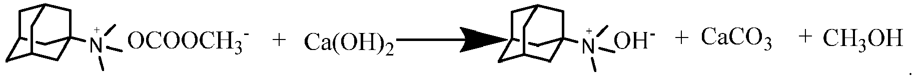

A reaction mechanism of the present invention is as follows.

In step S1, the dimethyladamantanamine reacts with the dimethyl carbonate to produce the adamantyltrimethylammonium carbonate monomethyl ester salt first; in step S1, the salification is carried out under the helium atmosphere, on one hand, the helium is an inert gas, which is conductive to preventing an oxidation reaction between the dimethyladamantanamine and the adamantyltrimethylammonium carbonate monomethyl ester salt; and on the other hand, the helium has a high heat conductivity coefficient, which is conducive to accelerating heat exchange in a salification system, preventing local overheating, inhibiting decomposition between the dimethyladamantanamine and the adamantyltrimethylammonium carbonate monomethyl ester salt, reducing an impurity content in a product, improving a product quality, exerting a template function of the product during molecular sieve synthesis better, and increasing a specific surface area and a crystallinity of a downstream molecular sieve.

In step S2, the adamantyltrimethylammonium carbonate monomethyl ester salt is ionized to form adamantyltrimethylammonium cations and carbonate monomethyl anions, and a small amount of calcium hydroxide is dissolved to form calcium ions and hydroxide ions; under the reaction conditions, the carbonate monomethyl anions are hydrolyzed to produce methanol and carbonate ions, the carbonate ions are combined with the calcium ions to form calcium carbonate which is precipitated, the methanol is volatilized and removed in a form of gas phase, and the remaining adamantyltrimethylammonium cations are combined with the hydroxide ions to form the adamantyltrimethylammonium hydroxide; in step S2, a chemical balance of hydrolysis of the carbonate monomethyl anions is a key to determine a reaction conversion rate, the nitrogen is blown from the lower part of the liquid level during hydrolysis in step S2 in the present invention, the nitrogen used as the inert gas may prevent the product from being oxidized, which is also conductive to preventing an alkaline product from absorbing carbon dioxide in the air, the methanol produced by hydrolysis of the carbonate monomethyl anions may be removed in time by blowing the nitrogen from the lower part of the liquid level, which promotes a forward movement of the chemical balance of hydrolysis of the carbonate monomethyl anions, and simultaneously promotes combination of the carbonate ions and the calcium ions to form the calcium carbonate, and an amount of the calcium hydroxide can be reduced by blowing the nitrogen, thus reducing a content of the calcium ions in the product, improving a product purity, and being conductive to increasing the specific surface area and the crystallinity of the downstream molecular sieve product.

By using the following preferred technical solutions, better reaction speed and yield may be obtained.

In step S1:
an initial absolute pressure of the helium before the heating for reaction is 0.15 MPa to 0.2 MPa. By using the above technical solutions, a proper initial pressure of the helium is selected in the step of salification, which is conductive to promoting occurrence of the salification, improving a conversion rate and a yield, and reducing a cost.

A molar ratio of the dimethyladamantanamine to the dimethyl carbonate in the quaternized mixture is 1: 3 to 10; by using the above technical solutions, an amount of the dimethyl carbonate is increased, and on one hand, the dimethyl carbonate is used as a salifying reagent; and on the other hand, the dimethyladamantanamine is dissolved in the dimethyl carbonate, the dimethyl carbonate is used as a reaction solvent, without adding other solvents, and since the product formed by the salification in step S1 is insoluble in the dimethyl carbonate, and the product is precipitated in a solid form, excess dimethyl carbonate may be recycled, which is conductive to reducing a production cost.

Certainly, as an alternative solution, it is found by the applicant that, in step S1, polar acetonitrile may also be used as the reaction solvent, which is conductive to improving a salification rate and shortening a reaction time. Specifically, in step S1, a molar ratio of the dimethyladamantanamine to the dimethyl carbonate in the quaternized mixture is 1: (1 to 3); and the acetonitrile is added as the reaction solvent, and an amount of the acetonitrile is 2 times to 5 times an amount of the dimethyl carbonate. The polar acetonitrile is used as the reaction solvent, a small amount of salified product is dissolved in the acetonitrile, and the acetonitrile may be concentrated and removed to recover the salified product dissolved in the acetonitrile.

Preferably, the dimethyladamantanamine is prepared by the following method of: weighing adamantanamine, adding water with a weight no less than 2 times a weight of the adamantanamine, adding formic acid and formaldehyde, stirring the mixture, heating the mixture to 60°C to 80°C for reaction for no less than 240 minutes, and taking an organic phase to obtain the dimethyladamantanamine; and more preferably, a molar ratio of the adamantanamine to the formic acid and the formaldehyde is 1: (2.4 to 2.8): (2.1 to 2.5), and a reaction formula is as follows:

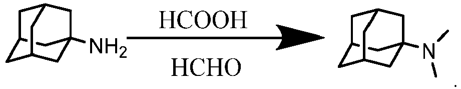

By using the above technical solutions, the adamantanamine is methylated with the formic acid and the formaldehyde to form the dimethyladamantanamine, and then the adamantyltrimethylammonium hydroxide is prepared through salification and hydrolysis, which avoids use of the sodium hydroxide, and is conductive to reducing a content of metal ions in the product.

Preferably, after the reaction in step S1 is completed, the reaction solution is filtered, a filter cake is dried in a vacuum state, and a product is recovered to obtain the adamantyltrimethylammonium carbonate monomethyl ester salt. By using the above technical solutions, the salified product in step S1 is dried in a vacuum state, which is conductive to preventing the salified product from being exposed to the air to be oxidized, thus reducing impurities and improving a product purity.

In step S2:
an amount of water used to dissolve the adamantyltrimethylammonium carbonate monomethyl ester salt is that 1 mol of adamantyltrimethylammonium carbonate monomethyl ester salt is corresponding to 800 ml to 1,500 ml of water. By using the above technical solutions, a proper amount of water is used in step S2, so that a concentration of the product is in a proper range, which is conductive to improving the product purity and the product quality.

A molar ratio of the adamantyltrimethylammonium carbonate monomethyl ester salt to the calcium hydroxide is 1: (1.01 to 1.03). By using the above technical solutions, a better raw material feeding ratio is selected in step S2, and the amount of the calcium hydroxide is reduced while maintaining a proper reaction yield, which is conductive to reducing the impurity content, and improving the product purity and the product quality.

Preferably, after the reaction in step S2, the temperature is reduced to room temperature under a nitrogen atmosphere, suction filtering is carried out, a filter cake is washed with water, and a filtrate is taken to obtain the aqueous quaternary ammonium base solution. By using the above technical solutions, after the reaction in step S2, the temperature is reduced under the nitrogen atmosphere, which is conductive to preventing oxidation, avoiding an alkaline material from absorbing carbon dioxide in the air, reducing impurities, and improving the product purity and the product quality.

The present invention aims to provide an aqueous quaternary ammonium base solution prepared by the preparation method of the adamantyltrimethylammonium hydroxide provide above in a second aspect, with characteristics of less impurities, high product purity and good quality.

The present invention has the beneficial effects as follows.

It is found by the inventor in practice that, during preparation of the adamantyltrimethylammonium carbonate monomethyl ester salt by the reaction between the dimethyl carbonate and the dimethyladamantanamine, when a reaction temperature is too low, a reaction yield is low, so that the reaction temperature generally exceeds 130°C, under this temperature condition, the product and the raw material are easy to decompose to produce impurities, thus affecting the product quality, increasing a chromaticity of the product, being not conducive to exerting a template function of the adamantyltrimethylammonium hydroxide as a template agent to produce a SSZ-13 molecular sieve, and affecting a performance of the SSZ-13 molecular sieve, and if the SSZ-13 molecular sieve with the same performance needs to be prepared, an amount of the template agent should be increased; during hydrolysis with the calcium hydroxide, in order to improve a hydrolysis effect of carbonate and a yield , it is generally necessary to increase the amount of the calcium hydroxide, and the molar ratio of the calcium hydroxide to the adamantyltrimethylammonium carbonate monomethyl ester salt is generally no less than 1.4; and the amount of the calcium hydroxide is large, so that the content of the calcium ions in the product is increased, and the chromaticity is increased, thus being not conducive to exerting the template function of the adamantyltrimethylammonium hydroxide as the template agent to produce the SSZ-13 molecular sieve.

On this basis, the present application mainly improves the reaction yield through the following improvements, and improves the product purity and the product quality at the same time without affecting the reaction yield.
(1) Improvement of product yield:
   In step S1, the reaction rate, the conversion rate and the yield are improved and the cost is reduced by selecting the proper initial pressure of the salification;
   in step S1, the salification is carried out under the helium atmosphere, and in step S2, the nitrogen is blown for reaction, thus effectively improving the reaction yield;
   by controlling the ratio of the raw materials for reaction in step S1 and step S2, a higher reaction yield may be obtained; and
   by controlling technological conditions in step S1 and step S2, a higher reaction yield may be obtained.
(2) Improvement of product quality:
   In step S1, during preparation of the raw material dimethyladamantanamine, the use of the sodium hydroxide is avoided, which is conductive to reducing the content of the metal ions in the product, and improving the product quality;
   in step S1, the salification is carried out under the helium atmosphere, in step S2, the hydrolysis is carried out under the condition of blowing the nitrogen, and the two reactions jointly reduce the amount of the calcium hydroxide, which is conductive to preventing decomposition and oxidation of the product, reducing the content of the impurities such as calcium ions, oxides and decomposed substances in the product, and improving the chromaticity, the product purity and the product quality.

There is no hydrolysis in step S1, and hydrolysis and precipitation in step S2 are carried out at the same time, which can effectively reduce the impurities, and realize better chromaticity and better template effect.

In step S1 and step S2, selection of proper salified product recovery technology and hydrolysis product recovery technology is conducive to preventing oxidation, reducing the impurity content and improving the product quality.

The technical solutions and effects of the present invention are explained and verified in detail with reference to the following examples.

### DETAILED DESCRIPTION

In the following examples, adamantanamine refers to 1-adamantanamine, used water is deionized water, with a conductivity no more than 20 µs/cm, and dimethyladamantanamine is prepared by the following method.

3,025 g of adamantanamine is weighted and added into a 20 L reactor, added with 6,725 g of water, stirred at a revolving speed of 100 rpm, added with 2,392 g of formic acid and 3,730 g of formaldehyde (with a mass concentration of 37%, the rest is water), and heated to 70°C for reaction for 360 minutes. A byproduct carbon dioxide is removed from the reaction system in a form of bubble and cooled to room temperature, and a pH value is adjusted to be greater than 12 with 30% liquid alkali (aqueous NaOH solution). The reaction solution is stood for 2 hours for layering, and an organic phase is taken and dried with anhydrous sodium sulfate, which is shown as the dimethyladamantanamine by GC, so that the dimethyladamantanamine is obtained, with a yield of 98.2%. A reaction formula is as follows.

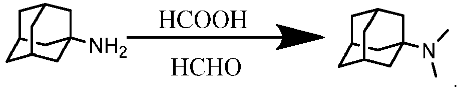

### Example 1:

A preparation method of adamantyltrimethylammonium hydroxide comprised the following steps.

S1. Salification: 169.3 g of dimethyladamantanamine was weighed and added with 900 g of dimethyl carbonate, and mixed evenly to obtain a quaternized mixture. The quaternized mixture was transferred into a 2 L high-pressure reactor, vacuumized until an absolute pressure was lower than 0.005 MPa, filled with helium until an absolute pressure in the reactor was 0.05 MPa, sealed, heated to 150°C under a helium sealed atmosphere, and reacted for 6 hours. The temperature was reduced to room temperature, and then discharging was carried out, the reaction solution was filtered, a filter cake was dried under -0.092 MPa at 60°C for 180 minutes, and a product was recovered to obtain adamantyltrimethylammonium carbonate monomethyl ester salt. A reaction formula was as follows.

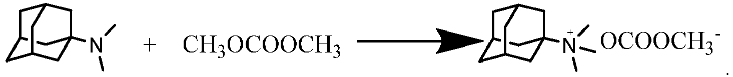

S2. Hydrolysis: 215.4 g of adamantyltrimethylammonium carbonate monomethyl ester salt prepared in step S1 was dissolved in 960 mL of water, transferred into a 2 L three-necked bottle, and added with 61.8 g of calcium hydroxide. A stirring device was mounted at a middle opening of the three-necked bottle, a nitrogen pipe and an h-shaped glass water separator were mounted at side openings on two sides of the three-necked bottle, and a spherical condenser pipe was mounted at an upper end of the glass water separator. Nitrogen was blown in from a lower part of a liquid level through the nitrogen pipe, with a nitrogen flow rate of 60 mL/min, and the three-necked bottle was heated in oil bath at 108°C. Byproduct methanol was separated from the three-necked bottle with the nitrogen, condensed, and then flowed back into the glass water separator, and a condensate could be discharged through a valve of the glass water separator. In an early stage of the reaction, more methanol and more condensate were produced by the hydrolysis. With the progress of the reaction, hydrolysis of monomethyl carbonate ions was gradually finished, and less methanol was produced. After reaction for 150 minutes, no methanol was removed, and the reaction was stopped. The temperature was reduced to room temperature under a nitrogen atmosphere, suction filtration was carried out, a filter cake was rinsed with 100 mL of water, and a filtrate was taken to obtain an aqueous quaternary ammonium base solution.

A reaction formula was as follows.

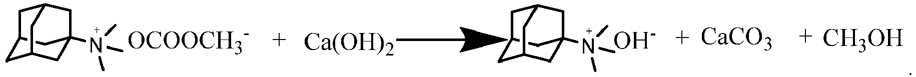

### Example 2

Example 2 was different from Example 1 in that the initial absolute pressure of the helium before heating in step S1 of Example 2 was 0.2 MPa. Others are consistent with those of Example 1.

### Example 3

Example 3 was different from Example 2 in that, in step S2 of Example 3, a molar ratio of calcium hydroxide to adamantyltrimethylammonium carbonate monomethyl ester salt was reduced to 1.01: 1. Others are consistent with those of Example 2.

### Example 4

Example 4 was different from Example 3 in that, in step S1 of Example 4, a molar ratio of dimethyladamantanamine to dimethyl carbonate was 1: 1, acetonitrile which was 5 times the volume of the dimethyl carbonate was used as a reaction solvent, suction filtration was carried out first after the reaction in step S1, a filtrate was concentrated in vacuum to recover a small amount of salt dissolved in the acetonitrile, and solid phase parts were combined, and then dried. Others are consistent with those of Example 3.

### Examples 5-8

Examples 5 to 8 were different from Example 3 in that, molar ratios of raw materials in Examples 5 to 8 were different. Others are consistent with those of Examples. The molar ratios of the raw materials in Examples 5 to 8 were shown in Table 1.

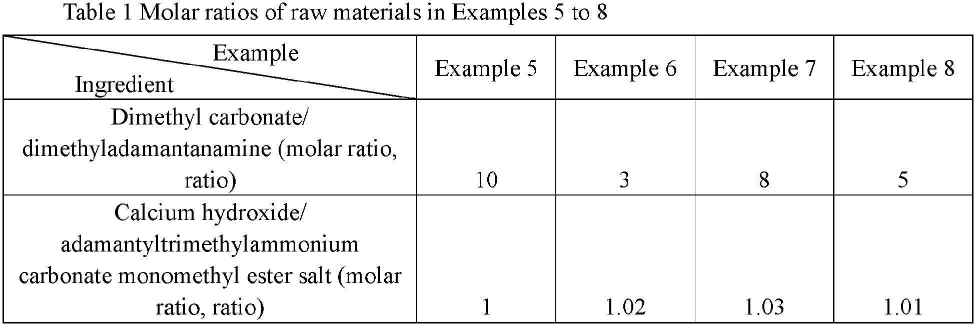

### Examples 9-12

Examples 9 to 12 were different from Example 3 in that, process parameters of the steps in Examples 9 to 12 were different. Others are consistent with those of Example 3. The process parameters of the steps in Examples 9 to 12 were shown in Table 2.

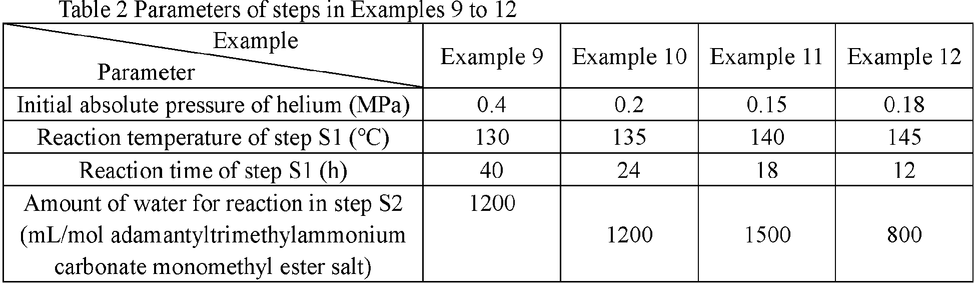

### Comparative Examples

### Comparative Example 1

Comparative Example 1 was different from Example 1 in that, a reaction atmosphere before the heating in step S1 of Comparative Example 1 was an atmospheric pressure air atmosphere. Others are consistent with those of Example 1.

### Comparative Example 2

Comparative Example 2 was different from Example 1 in that, there was no blowing of nitrogen in step S2 of Comparative Example 2. Others are consistent with those of Example 1.

### Comparative Example 3

Comparative Example 3 was different from Comparative Example 2 in that, a molar ratio of adamantyltrimethylammonium carbonate monomethyl ester salt to calcium hydroxide in step S2 of Comparative Example 3 was 1: 1.5. Others are consistent with those of Comparative Example 2.

### Comparative Example 4

Comparative Example 4 was different from Comparative Example 3 in that, a reaction atmosphere before the heating in step S1 of Comparative Example 4 was an atmospheric pressure air atmosphere. Others are consistent with those of Comparative Example 3.

### Application Examples

### Application Example 1

The aqueous adamantyltrimethylammonium hydroxide solution prepared in Comparative Example 4 was used as a template agent (represented by R), 50% aqueous silica sol solution was used as a silicon source, aluminum sulfate was used as an aluminum source, and sodium hydroxide was used as an alkali source. Mixing was carried out according to a molar ratio that R: NaOH: Al₂O₃: SiO₂: H₂O = 0.05: 0.4: 0.04: 1: 40. 0.5 g of seed crystal (SSZ-13 molecular sieve raw powder) was added into every 100 g of gel, and stirred at 30°C for 2 hours to form gel. The gel was put into a stainless steel high pressure reactor, crystallized at 175°C for 80 hours, filtered, washed, dried, baked at 550°C for 4 hours, and sent as a sample for XRD and specific surface area detection. XRD showed the SSZ-13 molecular sieve, and the specific surface area was 418 m²/g.

### Application Example 2

Application Example 2 was different from Application Example 1 in that, the aqueous adamantyltrimethylammonium hydroxide solution prepared in Example 3 was used as the template agent (represented by R) in Application Example 2. Others are consistent with those of Application Example 1. For the product prepared in Application Example 2, XRD showed the SSZ-13 molecular sieve, and the specific surface area was 683 m²/g.

### Performance detection

A yield, a chromaticity and a content of calcium ions of the aqueous quaternary ammonium base solution prepared in Examples 1 to 12 and Comparative Examples 1 to 4 were measured respectively. Specific methods were as follows.
(1) Calculation of yield: a product content of the aqueous quaternary ammonium base solution was detected, and a yield in step S2 was calculated. Experimental results were shown in Table 3. A detection method of the product content of the aqueous quaternary ammonium base solution was as follows: 15 g of sample solution (accurate to 0.0002 g) was weighed, diluted to a 100 mL volumetric flask, and shaken evenly to prepare a sample solution. 10 mL of sample solution was weighed, injected into a 250 mL triangular bottle with a stopper, added with 10.00 mL of barium chloride solution with a concentration of 100 g/L, added with 2 drops to 3 drops of phenolphthalein indicator (obtained by dissolving 0.1 g of phenolphthalein with a concentration of 10 g/L in ethanol and diluting to 100 mL with ethanol), and under stirring of a magnetic stirrer, titrated with a standard hydrochloric acid titration solution with a concentration of 0.1000 mol/L until a red color was faded. A volume of the consumed standard titration solution was recorded as V1. A content (X1%) represented by mass percentage was calculated according to the following formula: X1%=V1c×211.35, wherein V1 was the volume of the standard hydrochloric acid solution consumed for measuring the sample solution, in a unit of ml; c was a molar concentration of the standard hydrochloric acid solution, in a unit of mol/L; m was a mass of the sample, in a unit of g; and 211.35 was a molar mass of 1-adamantyltrimethylammonium hydroxide, in a unit of g/mol. The yield in step S1 was calculated during the experiment. Experimental results were shown in Table 3.
(2) Detection of chromaticity: with reference to GB/T9282.1-2008 "Clear Liquids - Estimation of Color by the Platinum - Cobalt Scale - Part 1: Visual Method", the chromaticity of the product was detected. Experimental results were shown in Table 3.
(3) Measurement of content of calcium ions: with reference to GB/T11446.5-2013 "Test Method for Measuring Trace Metals in Electronic Grade Water by Atomic Absorption Spectrophotometry", the content of calcium ions in the product was measured. Experimental results were shown in Table 3.

**Table 3 Performance comparison table of products prepared in different examples**

| Experiment number | Yield in step S1 (%) | Yield in step S2 (%) | Chromaticity (Hazen) | Content of calcium ions (mg/ L) |
|---|---|---|---|---|
| Example 1 | 92.3 | 98.2 | 58 | 12 |
| Example 2 | 96.5 | 97.8 | 45 | 9 |
| Example 3 | 96.9 | 97.6 | 38 | 2 |
| Example 4 | 98.6 | 94.3 | 76 | 16 |
| Example 5 | 97.2 | 95.1 | 52 | 7 |
| Example 6 | 96.1 | 98.5 | 34 | 3 |
| Example 7 | 98.4 | 98.1 | 31 | 2 |
| Example 8 | 98.2 | 97.6 | 36 | 1 |
| Example 9 | 85.6 | 94.7 | 147 | 8 |
| Example 10 | 97.5 | 98.4 | 32 | 2 |
| Example 11 | 98.1 | 98.2 | 34 | 3 |
| Example 12 | 98.3 | 97.9 | 29 | 2 |
| Comparative Example 1 | 81.4 | 98.6 | 314 | 15 |
| Comparative Example 2 | 92.1 | 84.5 | 273 | 18 |
| Comparative Example 3 | 91.9 | 97.8 | 351 | 46 |
| Comparative Example 4 | 81.7 | 98.1 | 392 | 45 |

### Analysis:

The atmospheric pressure air atmosphere is used before heating in step S1 of Comparative Example 1. The yield in step S1 is low, the chromaticity of the hydrolysis product is high, and the product should contain a certain amount of impurities. There is no blowing of nitrogen in step S2 of Comparative Example 2. Under the same reaction time, the yield in step S2 is low, and the chromaticity is high. Based on Comparative Example 2, the amount of the calcium hydroxide is increased in Comparative Example 3, and the yield in step S2 is significantly improved, but the chromaticity of the product and the content of calcium ions in the product are increased. Step S1 of Comparative Example 4 is carried out under the air atmosphere , there is no blowing of nitrogen in step S2, the amount of the calcium hydroxide is increased, the chromaticity of the product prepared is large, and the content of calcium ions is high.

It can be seen from comparison between the experimental results of Example 1 and the experimental results of Comparative Examples 1 to 4 that: during preparation of the adamantyltrimethylammonium hydroxide, the salification in step S1 is carried out under the helium atmosphere, and the nitrogen is blown in step S2, which is conductive to improving a reaction yield, reducing a chromaticity and an impurity content, and improving a product quality.

Comparing the experimental results of Example 1 with the experimental results of Example 2, a better initial pressure of helium is used in Example 2, the yield is increased, the chromaticity of the product is reduced, and the impurity content is reduced, thus being conductive to improving the product quality.

Comparing the experimental results of Example 2 with the experimental results of Example 3, the amount of the calcium hydroxide is appropriately reduced in Example 3, the content of calcium ions in the product is reduced, and the chromaticity is slightly reduced.

Comparing the experimental results of Example 3 with the experimental results of Example 4, the acetonitrile is used as the reaction solvent in Example 4, the yield in step S 1 is higher, the yield in step S2 is slightly reduced, and the chromaticity is slightly increased.

Examples 5 to 8 are comparative tests of different ratios of reaction raw materials. On the whole, Example 7 has a higher reaction yield, a better chromaticity of the product, a lower content of calcium ions, and a higher product quality.

Examples 9 to 12 are comparative tests of different process conditions, wherein the initial pressure in Example 9 is excessively high, the yield in step S 1 is low, and the chromaticity of the product is good. Examples 10 to 12 select better process conditions, with a higher reaction yield, a better chromaticity of the product, a lower content of calcium ions, and a higher product quality.

Comparing the experimental results of Application Example 1 with the experimental results of Application Example 2, compared with Comparative Example 4, the specific surface area of the molecular sieve product prepared by using the adamantyltrimethylammonium hydroxide prepared in Example 3 as the template agent is increased in detail, and the product quality is improved.

## Claims

1. A preparation method of adamantyltrimethylammonium hydroxide, comprising the following steps of:
S1, salification: weighing dimethyladamantanamine, adding dimethyl carbonate, evenly mixing the mixture to prepare a quaternized mixture, under a helium sealing atmosphere, heating the quaternized mixture to 135°C to 150°C for reaction for no less than 6 hours, and recovering a product to obtain adamantyltrimethylammonium carbonate monomethyl ester salt; wherein a molar ratio of the dimethyladamantanamine to the dimethyl carbonate in the quaternized mixture is 1:1 to 10; and
S2, hydrolysis: dissolving the adamantyltrimethylammonium carbonate monomethyl ester salt into water, adding calcium hydroxide, wherein a molar ratio of the adamantyltrimethylammonium carbonate monomethyl ester salt to the calcium hydroxide is 1:1 to 1.045, blowing nitrogen from a lower part of a liquid level, heating the mixture until a boiling state is reached, simultaneously removing a byproduct methanol to form the adamantyltrimethylammonium hydroxide, and recovering a product to prepare an aqueous quaternary ammonium base solution.

2. The preparation method of the adamantyltrimethylammonium hydroxide according to claim 1, wherein an initial absolute pressure of the helium before the heating for reaction in step S1 is 0.15 MPa to 0.2 MPa.

3. The preparation method of the adamantyltrimethylammonium hydroxide according to claim 1, wherein a molar ratio of the dimethyladamantanamine to the dimethyl carbonate in the quaternized mixture is 1:3 to 10.

4. The preparation method of the adamantyltrimethylammonium hydroxide according to claim 1, wherein a molar ratio of the dimethyladamantanamine to the dimethyl carbonate in the quaternized mixture is 1:1 to 3; and acetonitrile is added as a reaction solvent in step S1, and an amount of the acetonitrile is 2 times to 5 times a volume of the dimethyl carbonate.

5. The preparation method of the adamantyltrimethylammonium hydroxide according to claim 3, wherein after the reaction in step S1 is completed, the reaction solution is filtered, a filter cake is dried in a vacuum state, and a product is recovered to obtain the adamantyltrimethylammonium carbonate monomethyl ester salt

6. The preparation method of the adamantyltrimethylammonium hydroxide according to claim 1, wherein the dimethyladamantanamine is prepared by the following method of: weighing adamantanamine, adding water with a weight no less than 2 times a weight of the adamantanamine, adding formic acid and formaldehyde, stirring the mixture, heating the mixture to 6o°C to 8o°C for reaction for no less than 240 minutes, and taking an organic phase to obtain the dimethyladamantanamine.

7. The preparation method of the adamantyltrimethylammonium hydroxide according to claim 1, wherein in step S2, an amount of water used to dissolve the adamantyltrimethylammonium carbonate monomethyl ester salt is that 1 mol of adamantyltrimethylammonium carbonate monomethyl ester salt is corresponding to 800 ml to 1,500 ml of water.

8. The preparation method of the adamantyltrimethylammonium hydroxide according to claim 1, wherein in step S2, the molar ratio of the adamantyltrimethylammonium carbonate monomethyl ester salt to the calcium hydroxide is 1:1.01 to 1.03.

9. The preparation method of the adamantyltrimethylammonium hydroxide according to claim 1, wherein after the reaction in step S2, the temperature is reduced to room temperature under a nitrogen atmosphere, suction filtering is carried out, a filter cake is washed with water, and a filtrate is taken to obtain the aqueous quaternary ammonium base solution.

## Patentansprüche

1. Verfahren zur Herstellung von Adamantyltrimethylammoniumhydroxid, umfassend die folgenden Schritte:
S1: Salzbildung: Wiegen von Dimethyladamantanamin, Hinzufügen von Dimethylcarbonat, gleichmäßiges Mischen des Gemisches, um ein quaternisiertes Gemisch herzustellen, unter einer Helium-Abdichtungsatmosphäre, Erhitzen des quaternisierten Gemisches auf 135 °C bis 150 °C zur Reaktion für nicht weniger als 6 Stunden, Gewinnen eines Produkts, um Adamantyltrimethylammoniumcarbonat-Monomethylester-Salz zu erhalten; wobei ein molares Verhältnis des Dimethyladamantanamins zu dem Dimethylcarbonat in dem quaternisierten Gemisch 1: 1 bis 10 ist; und
S2: Hydrolyse: Auflösen des Adamantyltrimethylammoniumcarbonat-Monomethylester-Salzes in Wasser, Hinzufügen von Calciumhydroxid, wobei das Molverhältnis des Adamantyltrimethylammoniumcarbonat-Monomethylester-Salzes zum Calciumhydroxid 1: 1 bis 1, 045 beträg, Einblasen von Stickstoff aus einem unteren Teil eines Flüssigkeitsspiegels, Erhitzen des Gemisches, bis zum Erreichen eines Siedezustandes, gleichzeitiges Entfernen eines Nebenprodukts Methanol, um das Adamantyltrimethylammoniumhydroxid zu bilden, und Gewinnen eines Produkts, um eine wässrige Lösung einer quaternären Ammoniumbase herzustellen.

2. Verfahren zur Herstellung von Adamantyltrimethylammoniumhydroxid nach Anspruch 1, wobei der anfängliche absolute Druck des Heliums vor dem Erhitzen zur Reaktion in Schritt S1 0,15 MPa bis 0,2 MPa beträgt.

3. Verfahren zur Herstellung von Adamantyltrimethylammoniumhydroxid nach Anspruch 1, wobei das molare Verhältnis von Dimethyladamantanamin zu Dimethylcarbonat in dem quaternisierten Gemisch 1: 3 bis 10 beträgt.

4. Verfahren zur Herstellung von Adamantyltrimethylammoniumhydroxid nach Anspruch 1, wobei das molare Verhältnis von Dimethyladamantanamin zu Dimethylcarbonat in dem quaternisierten Gemisch 1:1 bis 3 beträgt; wobei Acetonitril als Reaktionslösungsmittel in Schritt S1 zugegeben wird, und wobei die Menge des Acetonitrils das 2- bis 5-fache des Volumens des Dimethylcarbonats beträgt.

5. Verfahren zur Herstellung von Adamantyltrimethylammoniumhydroxid nach Anspruch 3, wobei nach Beendigung der Reaktion in Schritt S1 die Reaktionslösung filtriert wird, ein Filterkuchen im Vakuum getrocknet wird und ein Produkt gewonnen wird, um das Adamantyltrimethylammoniumcarbonat-Monomethylester-Salz zu erhalten.

6. Verfahren zur Herstellung von Adamantyltrimethylammoniumhydroxid nach Anspruch 1, wobei das Dimethyladamantanamin durch das folgende Verfahren hergestellt wird: Wiegen von Adamantanamin, Hinzufügen von Wasser mit einem Gewicht von nicht weniger als dem 2-fachen des Gewichts des Adamantanamins, Hinzufügen von Ameisensäure und Formaldehyd, Rühren des Gemisches, Erhitzen des Gemisches auf 60°C bis 80°C zur Reaktion für nicht weniger als 240 Minuten und Entnehmen einer organischen Phase, um das Dimethyladamantanamin zu erhalten.

7. Verfahren zur Herstellung von Adamantyltrimethylammoniumhydroxid nach Anspruch 1, wobei in Schritt S2 die Menge an Wasser, die zum Lösen des Adamantyltrimethylammoniumcarbonat-Monomethylester-Salzes verwendet wird, so groß ist, dass 1 Mol Adamantyltrimethylammoniumcarbonat-Monomethylester-Salz 800 ml bis 1500 ml Wasser entspricht.

8. Verfahren zur Herstellung von Adamantyltrimethylammoniumhydroxid nach Anspruch 1, wobei in Schritt S2 das Molverhältnis des Adamantyltrimethylammoniumcarbonat-Monomethylester-Salzes zum Calciumhydroxid 1:1,01 bis 1,03 beträgt.

9. Verfahren zur Herstellung von Adamantyltrimethylammoniumhydroxid nach Anspruch 1, wobei nach der Reaktion in Schritt S2 die Temperatur unter einer Stickstoffatmosphäre auf Raumtemperatur gesenkt wird, eine Saugfiltration durchgeführt wird, ein Filterkuchen mit Wasser gewaschen wird und ein Filtrat entnommen wird, um die wässrige Lösung einer quaternären Ammoniumbase zu erhalten.

## Revendications

1. Procédé de préparation de l'hydroxyde d'adamantyltriméthylammonium :
comprenant les étapes suivantes :
S1, salification : peser de la diméthyladamantanamine ; ajouter du carbonate de diméthyle, mélanger uniformément le mélange pour préparer un mélange quaternaire ; sous une atmosphère hermétique d'hélium, chauffer le mélange quaternaire jusqu'à 135 °C à 150 °C pour réagir pendant au moins 6 heures, et récupérer un produit pour obtenir un sel d'ester monométhylique de carbonate d'adamantyltriméthylammonium ; dans lequel un rapport molaire de la diméthyladamantanamine au carbonate de diméthyle dans le mélange quaternaire est de 1 : 1 à 10 ; et
S2, hydrolyse : dissoudre le sel d'ester monométhylique de carbonate d'adamantyltriméthylammonium dans l'eau, ajouter de l'hydroxyde de calcium, un rapport molaire du sel d'ester monométhylique de carbonate d'adamantyltriméthylammonium à l'hydroxyde de calcium étant de 1 : 1 à 1,045, souffler de l'azote à partir d'une partie inférieure d'un niveau liquide ; chauffer le mélange jusqu'à ce qu'un état d'ébullition soit atteint; éliminer simultanément un sous-produit de méthanol pour former l'hydroxyde d'adamantyltriméthylammonium ; et récupérer un produit pour préparer une solution aqueuse à base d'ammonium quaternaire.

2. Procédé de préparation de l'hydroxyde d'adamantyltriméthylammonium selon la revendication 1, dans lequel une pression absolue initiale de l'hélium avant le chauffage pour la réaction à l'étape S1 est de 0,15 MPa à 0,2 MPa.

3. Procédé de préparation de l'hydroxyde d'adamantyltriméthylammonium selon la revendication 1, dans lequel un rapport molaire de la diméthyladamantanamine au carbonate de diméthyle dans le mélange quaternaire est de 1 : 3 à 10.

4. Procédé de préparation de l'hydroxyde d'adamantyltriméthylammonium selon la revendication 1, dans lequel un rapport molaire de la diméthyladamantanamine au carbonate de diméthyle dans le mélange quaternaire est de 1 : 1 à 3 ; et l'acétonitrile est ajouté comme solvant réactionnel à l'étape S1, et une quantité d'acétonitrile est de 2 à 5 fois un volume de carbonate de diméthyle.

5. Procédé de préparation de l'hydroxyde d'adamantyltriméthylammonium selon la revendication 3, dans lequel une fois que la réaction à l'étape S1 est complétée, la solution réactionnelle est filtrée, un gâteau de filtration est séché sous vide et un produit est récupéré pour obtenir le sel d'ester monométhylique de carbonate d' adamantyltriméthylammonium.

6. Procédé de préparation de l'hydroxyde d'adamantyltriméthylammonium selon la revendication 1, dans lequel la diméthyladamantanamine est préparée par le procédé suivant : peser de l'adamantanamine ; ajouter de l'eau avec un poids d'au moins 2 fois le poids d'adamantanamine, ajouter de l'acide formique et du formaldéhyde, agiter le mélange, chauffer le mélange jusqu'à 60°C à 80°C pour réagir pendant au moins 240 minutes, et prendre une phase organique pour obtenir la diméthyladamantanamine.

7. Procédé de préparation de l'hydroxyde d'adamantyltriméthylammonium selon la revendication 1, dans lequel à l'étape S2, une quantité d'eau utilisée pour dissoudre le sel d'ester monométhylique de carbonate d'adamantyltriméthylammonium est que 1 mol de sel d'ester monométhylique de carbonate d'adamantyltriméthylammonium correspond à 800 ml à 1 500 ml d'eau.

8. Procédé de préparation de l'hydroxyde d'adamantyltriméthylammonium selon la revendication 1, dans lequel à l'étape S2, le rapport molaire du sel d'ester monométhylique de carbonate d'adamantyltriméthylammonium à l'hydroxyde de calcium est de 1 : 1,01 à 1,03.

9. Procédé de préparation de l'hydroxyde d'adamantyltriméthylammonium selon la revendication 1, dans lequel après la réaction à l'étape S2, la température est réduite à température ambiante sous une atmosphère d'azote, un filtrage par aspiration est effectué, un gâteau de filtration est lavé avec de l'eau et un filtrat est collecté pour obtenir la solution aqueuse à base d'ammonium quaternaire.
